# EUROPEAN PATENT APPLICATION

(11) **EP 4 748 872 A1**
(43) Date of publication of application: **27.05.2026**
(21) Application number: 25216340.7
(22) Date of filing: 17.11.2025
(51) Int. Cl.: C08G 63/91, C08K 3/30, C08L 67/02, C08G 63/20, C08K 5/49

(54) **SUCCINIC ACID COMPOSITION, POLYESTER PREPARED THEREFROM, AND POLYESTER COMPOSITE MATERIAL**

(30) Priority: 26.11.2024 CN 202411701210
(71) Applicant: Zhuhai Kingfa Biomaterial Co., Ltd., Zhuhai, Guangdong 519050 (CN); Liaoning Kingfa Biomaterial Co., Ltd., Panjin City, Liaoning Province 124000 (CN); KINGFA SCI. & TECH. CO., LTD., Guangzhou, Guangdong 510663 (CN)
(72) Inventor: Lu, Changli, Zhuhai,Guangdong, 519050 (CN); Chen, Pingxu, Zhuhai,Guangdong, 519050 (CN); Ye, Nanbiao, Zhuhai,Guangdong, 519050 (CN); Zeng, Xiangbin, Zhuhai,Guangdong, 519050 (CN); Fu, Xuejun, Zhuhai,Guangdong, 519050 (CN); Zhang, Jialong, Zhuhai,Guangdong, 519050 (CN); Chen, Meiqi, Zhuhai,Guangdong, 519050 (CN); Ouyang, Chunping, Zhuhai,Guangdong, 519050 (CN); Yao, Yi, Zhuhai,Guangdong, 519050 (CN)
(74) Representative: Petraz, Gilberto Luigi

(57) **Abstract**

The present invention provides a succinic acid composition, a polyester prepared therefrom, and a polyester composite material. The succinic acid composition includes succinic acid, a compound containing a sulfate radical, and a phosphorus compound; a mass content of the sulfate radical in the succinic acid composition is 240-500 ppm; and a mass content of a phosphorus element in the succinic acid composition is 0.5-40 ppm. By compounding the compound containing the sulfate radical with the phosphorus compound at specific contents to prepare the polyester using the succinic acid composition as a raw material, not only can the production efficiency of the polyester be ensured to obtain the polyester with both a low acid number and a high viscosity, but also the corrosion of metal equipment caused by the polyester or a composition thereof during extrusion of the polyester composite material can be reduced to ensure the quality of the polyester composite material.

## Description

### TECHNICAL FIELD

The present invention belongs to the technical field of preparation of polyesters, and particularly relates to a succinic acid composition, a polyester prepared therefrom, and a polyester composite material.

### BACKGROUND

Poly(butylene succinate) (PBS) is produced by esterification and polycondensation reactions of succinic acid and 1,4-butanediol, and can be processed and obtained on common forming and processing equipment by adopting extrusion, injection molding, blow molding, spinning, vacuum forming, laminating, foaming, and other manners. Its products have a wide use range and have been mainly used in fields including the packaging field, such as cling films, courier bags, disposable tableware, and the like.

However, due to inherent performance defects, pure PBS resins are usually difficult to meet demands of an application end. Therefore, the PBS resins usually need to be blended and modified with other materials to be processed into polyester composite materials that meet the demands of the application end through thermal forming processes such as twin-screw extrusion. Since the PBS resins themselves contain a certain number of carboxyl functional groups and have poor thermal stability, the PBS resins are prone to decomposition under heat during processing to release acidic substances, and the acidic substances will corrode metal equipment. Therefore, for the polyester composite materials that need to be prepared and obtained through thermal forming and when thermal forming equipment includes metal components, for example, when a screw extruder is used for thermal forming and processing, severe corrosion of the screw extruder is easily caused after the polyester composite materials are produced by the screw extruder for a period of time. The corrosion of the screw extruder, on one hand, will weaken processing capabilities of the screw extruder, for example, decreasing shearing and dispersion capabilities, and on the other hand, will also result in the generation of foreign object spots on the polyester composite materials, thus resulting in reduced product quality.

Therefore, the development of a succinic acid composition having high reactivity and being capable of improving polyester production efficiency to obtain a polyester with both a low acid number and a high viscosity, and meanwhile, capable of reducing the corrosion of equipment caused by the polyester composite materials in a thermal forming process, is an urgent problem to be solved in the art.

### SUMMARY

In view of the shortcomings of the prior art, the purpose of the present invention is to provide a succinic acid composition, a polyester prepared therefrom, and a polyester composite material. The polyester prepared using the succinic acid composition as a raw material has high reactivity and high production efficiency, and the obtained polyester has a low acid number and a high viscosity. Furthermore, when the polyester composite material is prepared by a thermal forming process, the degree of corrosion of equipment is small.

To achieve the purpose, the present invention adopts the following technical solutions.

In a first aspect, the present invention provides a succinic acid composition, where the succinic acid composition includes succinic acid, a compound containing a sulfate radical, and a phosphorus compound; a mass content of the sulfate radical in the succinic acid composition is 240-500 ppm; and a mass content of a phosphorus element in the succinic acid composition is 0.5-40 ppm.

In the present invention, the compound containing the sulfate radical can improve the esterification efficiency of the succinic acid to improve the production efficiency of the polyester, and is conducive to obtaining the polyester with a low acid number and a high viscosity, and the polyester composite material, meanwhile, the polyester with a low acid number is also conducive to reducing the corrosion of equipment caused by the polyester during processing; the phosphorus compound is based on an electronic coordination effect, phosphorus atoms can form a structurally stable metal complex with metal ions in a manner of donating electron pairs, and the metal complex covering a metal surface can play a protective role on the metal to slow down the corrosion of metal equipment caused by a polyester material during processing; by compounding the compound containing the sulfate radical with the phosphorus compound at specific contents to prepare the polyester using the succinic acid composition as a raw material, not only can the production efficiency of the polyester be ensured to obtain the polyester with both a lower acid number and a whiter color, but also the corrosion of metal equipment caused by the polyester during preparation of the polyester composite material using the polyester as a base resin can be reduced to ensure the quality of the polyester composite material.

In the present invention, the mass content of the sulfate radical in the succinic acid composition is 240-500 ppm, and for example, may be 240 ppm, 245 ppm, 250 ppm, 255 ppm, 260 ppm, 265 ppm, 270 ppm, 275 ppm, 280 ppm, 285 ppm, 290 ppm, 295 ppm, 300 ppm, 305 ppm, 310 ppm, 315 ppm, 320 ppm, 325 ppm, 330 ppm, 335 ppm, 340 ppm, 345 ppm, 350 ppm, 355 ppm, 360 ppm, 365 ppm, 370 ppm, 375 ppm, 380 ppm, 385 ppm, 390 ppm, 395 ppm, 400 ppm, 405 ppm, 410 ppm, 415 ppm, 420 ppm, 430 ppm, 440 ppm, 450 ppm, 460 ppm, 470 ppm, 480 ppm, 490 ppm, 500 ppm, or a range between any of the above numerical values.

In the present invention, when the mass content of the sulfate radical is slightly low, the esterification efficiency is low, the residence time is long, and the obtained polyester has a high acid number and a low viscosity, thus also resulting in equipment corrosion; and when the mass content of the sulfate radical is slightly high, the corrosion to a metal is higher, the sulfate radical is easily dissolved in water to form a sulfuric acid solution in an aqueous environment, and released H⁺ ions make the solution acidic, thereby further intensifying metal corrosion.

In the present invention, the mass content of the phosphorus element in the succinic acid composition is 0.5-40 ppm, and for example, may be 0.5 ppm, 1 ppm, 2 ppm, 4 ppm, 6 ppm, 8 ppm, 10 ppm, 12 ppm, 14 ppm, 16 ppm, 18 ppm, 20 ppm, 22 ppm, 24 ppm, 26 ppm, 28 ppm, 30 ppm, 32 ppm, 34 ppm, 36 ppm, 38 ppm, 40 ppm, or a range between any of the above numerical values.

In the present invention, when the mass content of the phosphorus element is slightly low, severe equipment corrosion is caused during thermal processing; and when the content is slightly high, the activity of a catalyst is passivated, resulting in reduced catalytic efficiency, further resulting in prolonged polymerization reaction residence time, increased acid number of the polyester, yellowing in color, and also resulting in a certain degree of corrosion.

Preferably, the mass content of the sulfate radical in the succinic acid composition is 320-480 ppm, more preferably 370-420 ppm.

Preferably, the mass content of the phosphorus element in the succinic acid composition is 2-25 ppm, more preferably 5-20 ppm.

Preferably, the compound containing the sulfate radical includes sulfuric acid and/or a sulfate.

Preferably, the sulfate includes any one or at least two of sodium sulfate, magnesium sulfate, potassium sulfate, or ferric sulfate.

Preferably, the phosphorus compound includes any one or at least two of nucleic acid, phospholipid, diisooctyl phosphate, diethyl phosphate, triethyl phosphate, sodium dihydrogen phosphate, disodium hydrogen phosphate, phosphoric acid, phosphorous acid, or sodium phosphate. Preferably, a mass percentage content of the succinic acid in the succinic acid composition is ≥ 99.5%, and for example, may be 99.5%, 99.6%, 99.7%, 99.8%, 99.9%, or a range between any of the above numerical values; preferably, the mass percentage content of the succinic acid is ≥ 99.7%; and more preferably, the mass percentage content of the succinic acid is ≥ 99.8%.

In the present invention, the compound containing the sulfate radical and the phosphorus compound in the succinic acid composition may be a compound containing a sulfate radical and a phosphorus compound that are additionally added to obtain the succinic acid composition; and may also be some compounds remaining in the succinic acid during preparation of the succinic acid to form the succinic acid composition.

In the present invention, when the succinic acid composition is formed from some compounds remaining in the succinic acid during preparation of the succinic acid, the raw materials include a petroleum resource or a biomass resource.

In the present invention, a method for preparing the succinic acid composition includes a microbial fermentation method, a chemical synthesis method, or an additional addition and formulation method. When the microbial fermentation method or the chemical synthesis method is used, the compound containing the sulfate radical and the phosphorus compound may be derived from residues of any substances containing the sulfate radical or the phosphorus element added during preparation.

For example, when the succinic acid composition is prepared by the chemical synthesis method using the petroleum resource as a raw material, the compound containing the sulfate radical may be derived from electrolyte sulfuric acid used during catalytic hydrogenation preparation of maleic anhydride or from a sulfate additionally added as needed.

When the microbial fermentation method is used, the phosphorus compound may be derived from nucleic acid, phospholipid, and the like of a microorganism, and may also be derived from diisooctyl phosphate, diethyl phosphate, triethyl phosphate, sodium dihydrogen phosphate, disodium hydrogen phosphate, phosphoric acid, phosphorous acid, or sodium phosphate added during fermentation.

When the succinic acid composition is prepared by the microbial fermentation method using the biomass resource as a raw material, the compound containing the sulfate radical may be sulfuric acid added to adjust the pH value of a fermentation system or to acidify a succinate, or a sulfate additionally added as needed; and the phosphorus compound may be a phosphorus compound remaining during preparation of the succinic acid in the microbial fermentation method, and may also be an additionally added phosphorus compound.

In the present invention, the biomass resource includes a plant resource and/or an animal resource, and is preferably the plant resource.

In the present invention, the plant resource refers to a biomass resource capable of converting light energy of the sun into the form of starch or cellulose and storing the same through photosynthesis; the animal resource refers to a biomass resource that grows and develops by preying on plant bodies; and the plant animal or the animal resource further includes a product obtained by processing the plant animal or the animal resource.

In the present invention, exemplarily, the plant resource includes, but is not limited to, wood, rice straw, rice husk, rice bran, aged rice, corn, sugarcane, cassava, corn straw, cassava starch residue, bagasse, plant oil residue, buckwheat, soybean, food waste, and the like.

In the present invention, the biomass resource needs to be converted into a carbon source, then the carbon source is used as a raw material to prepare and obtain a succinic acid stock solution, and then the succinic acid stock solution is purified to obtain the succinic acid composition. A method for converting the biomass resource into the carbon source includes, but is not limited to, chemical treatment, physical treatment, biological treatment, and the like. Exemplarily, the chemical treatment may adopt acid treatment (for example, treatment with strong acids, such as sulfuric acid, nitric acid, hydrochloric acid, and phosphoric acid), alkali treatment, ammonia freezing, distillation and explosion treatment, solvent extraction treatment, supercritical fluid treatment, oxidizing agent treatment, and the like. The physical treatment may be crushing treatment, distillation and explosion treatment, microwave treatment, electron ray irradiation treatment, and the like. The biological treatment may be microbial treatment, enzymatic treatment, and the like.

In the present invention, the carbon source exemplarily includes, but is not limited to, hexoses, such as glucose, mannose, galactose, fructose, sorbitol, and tagatose; pentoses, such as arabinose, xylose, ribose, xylulose, and ribulose; and disaccharides or polysaccharides, such as pentosan, sucrose, starch, and cellulose. The carbon source preferably includes the glucose, the fructose, and the xylose, particularly preferably the glucose.

In the present invention, a microorganism used in the microbial fermentation method is capable of producing a dicarboxylic acid, and exemplarily includes: anaerobic bacteria, facultative anaerobic bacteria, aerobic bacteria, and the like; the anaerobic bacteria may be, for example, of the genus *Anaerobiospirillum* (US5143833A); the facultative anaerobic bacteria may be, for example, of the genus *Actinobacillus* (US5504004A), the genus *Escherichia* (US5770435A), and the like; and the aerobic bacteria may be, for example, of the genus *Corynebacterium* (JPH0 11-113588 communique or CN103183813A), where these documents are incorporated herein by reference. The aerobic bacteria, such as those of the genus *Corynebacterium,* are preferably used.

Exemplarily, taking the microbial fermentation method as an example, the method for preparing the succinic acid composition includes:
(1) using the biomass resource as a raw material to prepare and obtain a succinic acid fermentation solution by the microbial fermentation method (may adopt a conventional method, and for example, may refer to a method in CN118459331A for preparation); and filtering the succinic acid fermentation solution, adding an excess amount of calcium hydroxide to fully precipitate succinic acid, and performing filtration to obtain calcium succinate;
(2) mixing the calcium succinate obtained in the step (1) with concentrated sulfuric acid, and adjusting the pH value to 1.2-3 (for example, may be 1.2, 1.3, 1.5, 1.6, 1.8, 2, 2.1, 2.2, 2.3, 2.4, 2.5, 2.6, 2.7, 2.8, 2.9, 3, or a range between any of the above numerical values) to obtain a succinic acid stock solution with a succinic acid mass concentration of 65-80 g/L (for example, may be 65 g/L, 68 g/L, 70 g/L, 72 g/L, 75 g/L, 78 g/L, 80 g/L, or a range between any of the above numerical values);
(3) subjecting the succinic acid stock solution obtained in the step (2) to forward cross-current extraction with a phosphate ester extractant to obtain a succinic acid-loaded organic phase; and then, subjecting the succinic acid-loaded organic phase to reverse cross-current extraction with water to obtain an aqueous solution of succinic acid, where based on a volume of 1 L of the succinic acid stock solution, a mass of the phosphate ester extractant is 2-55 g (for example, may be 2 g, 5 g, 8 g, 10 g, 12 g, 15 g, 18 g, 20 g, 22 g, 24 g, 26 g, 28 g, 30 g, 32 g, 34 g, 36 g, 38 g, 40 g, 42 g, 44 g, 46 g, 48 g, 50 g, 52 g, 54 g, 55 g, or a range between any of the above numerical values); based on a volume of 1 L of the succinic acid-loaded organic phase, a mass of the water is 30-95 g (for example, may be 30 g, 32 g, 35 g, 38 g, 40 g, 42 g, 45 g, 48 g, 50 g, 52 g, 55 g, 58 g, 60 g, 62 g, 65 g, 68 g, 70 g, 72 g, 75 g, 78 g, 80 g, 82 g, 85 g, 88 g, 90 g, 92 g, 95 g, or a range between any of the above numerical values); the phosphate ester extractant includes at least one of diisooctyl phosphate, diethyl phosphate, or triethyl phosphate; and a number of times of the forward cross-current extraction and the reverse cross-current extraction is each independently ≥ 1 (for example, may be 1 time, 2 times, 3 times, 4 times, 5 times, 6 times, 7 times, 8 times, or a range between any of the above numerical values);
(4) allowing the aqueous solution of succinic acid obtained in the step (3) to pass through a cation exchange resin column at a flow rate of 0.5-2.5 BV/h (for example, may be 0.5 BV/h, 0.6 BV/h, 0.8 BV/h, 1 BV/h, 1.1 BV/h, 1.2 BV/h, 1.3 BV/h, 1.4 BV/h, 1.5 BV/h, 1.6 BV/h, 1.7 BV/h, 1.8 BV/h, 1.9 BV/h, 2 BV/h, 2.1 BV/h, 2.2 BV/h, 2.3 BV/h, 2.4 BV/h, 2.5 BV/h, or a range between any of the above numerical values), and performing elution with water with a mass 2-7 times (for example, may be 3 times, 4 times, 5 times, 6 times, or a range between any of the above numerical values) that of the aqueous solution of succinic acid to obtain a succinic acid effluent;
(5) sequentially subjecting the succinic acid effluent obtained in the step (4) to decolorization with activated carbon and filtration to obtain a succinic acid filtrate, where based on a volume of 1 L of the succinic acid effluent, a mass of the activated carbon is 0.5-10 g (for example, may be 0.5 g, 1 g, 2 g, 3 g, 4 g, 5 g, 5.2 g, 5.5 g, 5.8 g, 6 g, 6.2 g, 6.5 g, 6.8 g, 7 g, 7.2 g, 7.5 g, 7.8 g, 8 g, 8.5 g, 9 g, 9.5 g, 10 g, or a range between any of the above numerical values); and
(6) subjecting the succinic acid filtrate obtained in the step (4) to reduced-pressure distillation and cooling crystallization to obtain the succinic acid composition.

Preferably, a temperature of the decolorization with activated carbon is 60-85°C, and a time is 30-80 min.

In the present invention, the filtration includes filtration using an ultrafiltration membrane; and a pore size of the ultrafiltration membrane is 20-80 nm, and for example, may be 20 nm, 25 nm, 30 nm, 35 nm, 40 nm, 45 nm, 50 nm, 55 nm, 60 nm, 65 nm, 70 nm, 75 nm, or 80 nm, or a range between any of the above numerical values.

Preferably, a temperature of the filtration is 40-60°C, and for example, may be 40°C, 42°C, 44°C, 46°C, 48°C, 50°C, 52°C, 54°C, 56°C, 58°C, or 60°C, or a range between any of the above numerical values.

In the present invention, a temperature of the reduced-pressure distillation is 65-75°C, a pressure is -0.07 - -0.1 MPa, and the distillation is stopped when the content of succinic acid is greater than 15 wt%; and the concentrated succinic acid solution is subjected to the cooling crystallization at a low temperature (≤ 8°C) to obtain the succinic acid composition.

In a second aspect, the present invention provides a polyester, where raw materials for preparing the polyester include the succinic acid composition described in the first aspect.

Preferably, the polyester includes an aliphatic polyester.

Preferably, the polyester includes the following components:
based on a total molar amount of 100 mol% of component A, a dicarboxylic acid compound including the following components:
   a1, being 65-100 mol% of succinic acid and/or a derivative of an ester thereof, and
   a2, being 0-35 mol% of a C₆-C₁₄ dicarboxylic acid and/or a derivative of an ester thereof;
and component B: being 1,4-butanediol with a molar amount at least equal to that of the component A, where the succinic acid is the succinic acid composition described in the first aspect.

In the present invention, a molar ratio of the component B to the component A is (1-3):1, and for example, may be 1:1, 1.2:1, 1.5:1, 1.8:1, 2:1, 2.2:1, 2.5:1, 2.8:1, 3:1, or a range between any of the above numerical values, more preferably (1-2):1.

In the present invention, the phrase "the polyester includes the following components" means that a molecular structure of the polyester includes a structural unit derived from the component A and a structural unit derived from the component B; and the phrase "a dicarboxylic acid compound including the following components" means that in the molecular structure of the polyester, a dicarboxylic acid structural unit part includes a structural unit derived from the component a1 and a structural unit derived from the component a2.

In the present invention, 65-100 mol%, for example, may be 65 mol%, 66 mol%, 68 mol%, 70 mol%, 72 mol%, 74 mol%, 76 mol%, 78 mol%, 80 mol%, 82 mol%, 84 mol%, 86 mol%, 88 mol%, 90 mol%, 92 mol%, 94 mol%, 96 mol%, 98 mol%, 100 mol%, or a range between any of the above numerical values.

In the present invention, 0-35 mol%, for example, may be 0 mol%, 2 mol%, 4 mol%, 6 mol%, 8 mol%, 10 mol%, 12 mol%, 14 mol%, 16 mol%, 18 mol%, 20 mol%, 22 mol%, 24 mol%, 26 mol%, 28 mol%, 30 mol%, 32 mol%, 34 mol%, 35 mol%, or a range between any of the above numerical values.

In the present invention, when the component a1 is selected from a derivative of a succinic acid ester, the derivative of its ester is obtained using the succinic acid composition as a raw material.

Preferably, based on the total molar amount of 100 mol% of the component A, the dicarboxylic acid compound includes the following components:
a1, 72-82 mol% of the succinic acid and/or a derivative of an ester thereof, and
a2, 18-28 mol% of the C₆-C₁₄ dicarboxylic acid and/or a derivative of an ester thereof.

In the present invention, the derivative of the succinic acid ester includes an alkyl succinate; exemplarily, the alkyl succinate may be at least one of dimethyl succinate, diethyl succinate, di-n-propyl succinate, diisopropyl succinate, di-n-butyl succinate, diisobutyl succinate, di-tert-butyl succinate, di-n-pentyl succinate, diisopentyl succinate, and di-n-hexyl succinate; and the alkyl succinate may be an alkyl ester formed from succinic acid or an alkyl ester formed from succinic anhydride, and dimethyl succinate formed from succinic anhydride is preferably used.

In the present invention, the component a2 is selected from a C₆-C₁₄ linear dicarboxylic acid, for example, may be a C₆, C₇, C₈, C₉, C₁₀, C₁₁, C₁₂, C₁₃, or C₁₄ linear dicarboxylic acid, and exemplarily includes at least one of adipic acid, azelaic acid, sebacic acid, or brassilic acid, preferably the adipic acid and/or the sebacic acid.

In the present invention, a derivative of a C₆-C₁₄ dicarboxylic acid ester includes a C₆-C₁₄ dicarboxylic acid alkyl ester; exemplarily, the alkyl ester may be at least one of a dimethyl ester, a diethyl ester, a di-n-propyl ester, a diisopropyl ester, a di-n-butyl ester, a diisobutyl ester, a di-tert-butyl ester, a di-n-pentyl ester, a diisopentyl ester, and a di-n-hexyl ester; and the alkyl ester may be an alkyl ester formed from a diacid or an alkyl ester formed from a dianhydride.

In the present invention, a dicarboxylic acid or a derivative of an ester thereof may be used separately or used in the form of a mixture of two or more.

Preferably, in the polyester, a mass content of the sulfate radical is 90-210 ppm, and for example, may be 90 ppm, 95 ppm, 100 ppm, 105 ppm, 110 ppm, 115 ppm, 120 ppm, 125 ppm, 130 ppm, 135 ppm, 140 ppm, 145 ppm, 150 ppm, 155 ppm, 160 ppm, 165 ppm, 170 ppm, 175 ppm, 180 ppm, 185 ppm, 190 ppm, 195 ppm, 200 ppm, 205 ppm, 210 ppm, or a range between any of the above numerical values, further preferably 100-160 ppm.

Preferably, a mass content of a phosphide in the polyester is 0.1-15 ppm, and for example, may be 0.1 ppm, 0.2 ppm, 0.5 ppm, 1 ppm, 2 ppm, 3 ppm, 4 ppm, 5 ppm, 6 ppm, 7 ppm, 8 ppm, 9 ppm, 10 ppm, 11 ppm, 12 ppm, 13 ppm, 14 ppm, 15 ppm, or a range between any of the above numerical values, further preferably 1-10 ppm.

In the present invention, by using the succinic acid composition as a raw material, the polyester with both a low acid number and a high viscosity can be obtained. In the present invention, according to the standard DIN EN 12634-1998, the acid number of the polyester is ≤ 1.2 mg KOH/g, and for example, may be 0.4 mg KOH/g, 0.5 mg KOH/g, 0.6 mg KOH/g, 0.7 mg KOH/g, 0.8 mg KOH/g, 0.9 mg KOH/g, 1 mg KOH/g, 1.1 mg KOH/g, 1.2 mg KOH/g, or a range between any of the above numerical values. Further preferably, the acid number is ≤ 1 mg KOH/g; and more preferably, the acid number is ≤ 0.85 mg KOH/g.

Preferably, according to the standard GB/T 17931-1999, an intrinsic viscosity of the polyester is ≥ 1.4 dL/g, and for example, may be 1.4 dL/g, 1.45 dL/g, 1.5 dL/g, 1.55 dL/g, 1.6 dL/g, 1.65 dL/g, 1.7 dL/g, 1.75 dL/g, 1.8 dL/g, 1.9 dL/g, or a range between any of the above numerical values. Further preferably, the intrinsic viscosity is ≥ 1.52 dL/g; and more preferably, the intrinsic viscosity is ≥ 1.63 dL/g.

In the present invention, the polyester may be prepared by a conventional technical method in the art. Exemplarily, the method includes the following steps:
allowing the component A and the component B to undergo an esterification reaction at a temperature of 140-220°C and a pressure of 0.5-2 bar for 1-6 h to obtain an esterification product; allowing the esterification product to undergo a pre-polycondensation reaction at a temperature of 220-260°C and a pressure of 0.1-1 bar for 40-120 min to obtain a pre-polycondensation product; and then allowing the pre-polycondensation product to undergo a polycondensation reaction at a temperature of 230-280°C and a pressure of 1-3 mbar for 120-180 min, and performing slicing and drying to obtain the polyester.

In the present invention, raw materials for the esterification reaction further include a cross-linking agent, where the cross-linking agent includes at least one of tartaric acid, citric acid, malic acid, trimethylolpropane, trimethylolethane, pentaerythritol, polyether triol, glycerol, 1,3,5-benzenetricarboxylic acid, 1,2,4-benzenetricarboxylic acid, 1,2,4-benzenetricarboxylic anhydride, 1,2,4,5-benzenetetracarboxylic acid, or pyromellitic dianhydride; and based on a mass of 100 wt% of a finished product of the polyester, a mass percentage content of the cross-linking agent is 0.05-1 wt%.

In the present invention, the esterification reaction and/or the pre-polycondensation reaction further includes a catalyst; the catalyst may be a tin compound, an antimony compound, a cobalt compound, a lead compound, a zinc compound, an aluminum compound, or a titanium compound, more preferably the zinc compound, the aluminum compound, or the titanium compound, and most preferably the titanium compound; the titanium compound may be tetrabutyl titanate or tetraisopropyl titanate; and a total mass of the catalyst is 0.001-1 wt% of a mass of a polycondensation product.

In a third aspect, the present invention provides a polyester composite material, where the polyester composite material is a composite material obtained by extrusion processing; and the polyester composite material includes the polyester described in the second aspect.

Preferably, the polyester composite material further includes at least one of a polyhydroxyalkanoate, an inorganic filler, or an auxiliary agent.

Preferably, in parts by weight, the polyester composite material includes 40-65 parts of an aliphatic polyester, 20-30 parts of the polyhydroxyalkanoate, 5-25 parts of the inorganic filler, and 0-1 part of the auxiliary agent; and raw materials for preparing the aliphatic polyester include the succinic acid composition according to the first aspect, or the aliphatic polyester includes the polyester according to the second aspect.

In the present invention, 40-65 parts of the aliphatic polyester, for example, may be 40 parts, 42 parts, 44 parts, 46 parts, 48 parts, 50 parts, 52 parts, 54 parts, 56 parts, 58 parts, 60 parts, 62 parts, 64 parts, 65 parts, or a range between any of the above numerical values.

In the present invention, 20-30 parts of the polyhydroxyalkanoate, for example, may be 20 parts, 21 parts, 22 parts, 23 parts, 24 parts, 25 parts, 26 parts, 27 parts, 28 parts, 29 parts, 30 parts, or a range between any of the above numerical values.

In the present invention, 5-25 parts of the inorganic filler, for example, may be 5 parts, 6 parts, 8 parts, 10 parts, 12 parts, 14 parts, 16 parts, 18 parts, 20 parts, 22 parts, 24 parts, 25 parts, or a range between any of the above numerical values.

In the present invention, 0-1 part of the auxiliary agent, for example, may be 0 part, 0.1 part, 0.2 part, 0.4 part, 0.6 part, 0.8 part, 1 part, or a range between any of the above numerical values.

Preferably, the polyhydroxyalkanoate includes polylactic acid and/or poly-β-hydroxybutyrate.

Preferably, the inorganic filler includes at least one of talc powder, calcium carbonate, barium sulfate, montmorillonite, or kaolin.

In the present invention, the auxiliary agent includes, but is not limited to, an antioxidant; and the antioxidant includes, but is not limited to, antioxidant 1010.

In the present invention, when the polyester composite material is continuously produced in a twin-screw extruder for 168 hours, a wear percentage (η) of screws is ≤ 0.5%, preferably η ≤ 0.4%, more preferably η ≤ 0.29%, and particularly preferably η ≤ 0.13%.

The polyester and the polyester composite material of the present invention also have biodegradability.

For the present invention, if a substance or a substance mixture shows, as defined in DIN EN 13432, a biodegradation percentage degree of at least 90%, the substance or the substance mixture has the characteristic of "biodegradability".

Biodegradation usually leads to decomposition of a polyester or a polyester mixture within a reasonable inspection period. Degradation may occur through an enzymatic, hydrolytic, or oxidative pathway, and/or through exposure to electromagnetic radiation such as ultraviolet radiation, and is most commonly caused by exposure to microorganisms such as bacteria, yeast, fungi, or algae. By mixing the polyester with compost and storing the same for a specific period of time, the biodegradability may be quantified. For example, according to DIN EN 13432, during composting, air free of CO₂ is introduced into mature compost, and the compost is allowed to undergo a specific temperature process. Herein, the biodegradability is defined as a biodegradation percentage degree represented by a ratio of a net amount of CO₂ released by a sample (after subtracting an amount of CO₂ released by compost without the sample) to a maximum amount of CO₂ that can be released by the sample (calculated based on a carbon content in the sample).

Additionally, other methods for determining the biodegradability are described in ASTM D5338 and ASTM D6400.

The numerical ranges in the present invention include not only the above-mentioned enumerated point values but also any point values within the above-mentioned numerical ranges that are not enumerated, and due to space limitations and for the sake of simplicity, all specific point values included within the ranges are not exhaustively enumerated in the present invention.

Compared with the prior art, the present invention has the following beneficial effects.

According to the succinic acid composition provided by the present invention, by compounding the compound containing the sulfate radical with the phosphorus compound at specific contents to prepare the polyester using the succinic acid composition as a raw material, not only can the production efficiency of the polyester be ensured to obtain the polyester with both a low acid number and a high viscosity, but also the degree of corrosion of metal equipment caused by the polyester can be reduced to ensure the quality of the polyester material.

### DETAILED DESCRIPTION OF EMBODIMENTS

The technical solutions of the present invention are further described below through specific embodiments. Those skilled in the art should understand that examples described are merely to facilitate an understanding of the present invention and should not be regarded as specific limitations to the present invention.

Unless otherwise specified, raw materials used in the present invention are as follows:
1,4-butanediol: purchased from Xinjiang Markorchem Co., Ltd.;
commercially available succinic acid with a premium grade specification: purchased from Shandong Feiyang Chemical Co., Ltd., with a succinic acid content of 99.78%, a SO₄²⁻ content of 47 ppm, and a phosphorus element content of 0 ppm; glycerol: purchased from Aladdin;
tetrabutyl titanate: purchased from Jianyi Chemical Import and Export Co., Ltd.;
polylactic acid: PLA 3001D, purchased from Natureworks;
talc powder: HTPultra5 L, purchased from IMIFABI; and
calcium carbonate: OM-2T, purchased from Omya.

In the present invention, the content of SO₄²⁻ is determined by ion chromatography referring to the standard SN/T 2994-2011.

The content of a phosphorus element is measured according to the following procedure by referring to US EPA Method 3052:1996 and adopting ICP-OES analysis: weighing approximately 0.1 g of a succinic acid composition, adding 5 mL of nitric acid to completely immerse the succinic acid composition, then dropping 1.0 mL of hydrogen peroxide to carry out a reaction for 2 min, sealing a resulting mixture in a microwave digestion tank for digestion at 210°C for 3 h, cooling to room temperature, performing filtration with a 0.45 µm filter membrane, diluting the mixture to 50 mL with distilled water, and measuring the mixture by ICP-OES.

In the present invention, a method for measuring the mass content of succinic acid in the succinic acid composition refers to a method for determining the content of succinic acid in Part 4.3 of GB/T 34686-2017 for testing.

### Examples 1-6 and Comparative Examples 1-4

Examples 1-6 and Comparative Examples 1-4 respectively provide a succinic acid composition, which includes succinic acid, a sulfate, and a phosphorus compound; and in the succinic acid composition, mass contents of the succinic acid, the sulfate, and the phosphorus compound are shown in Table 1.

The mass content of the sulfate is represented by a mass content of a sulfate radical, and the mass content of the phosphorus compound is represented by a mass content of a phosphorus element.

The succinic acid compositions provided in Examples 1-3, Example 5, Example 6, and Comparative Example 2 are compositions formed from the sulfate and the phosphorus compound remaining in succinic acid during preparation of the succinic acid with the succinic acid. The succinic acid compositions provided in Example 4, Comparative Example 1, Comparative Example 3, and Comparative Example 4 are compositions formed from sodium sulfate and/or sodium dihydrogen phosphate additionally added into commercially available succinic acid (Shandong Feiyang Chemical Co., Ltd., with a premium grade specification) with the succinic acid, and remainders in the compositions include water and other impurity acids remaining during preparation of the succinic acid compositions.

### Comparative Example 5

This comparative example provides a succinic acid, which was purchased from Shandong Feiyang Chemical Co., Ltd., with a premium grade specification.

**Table 1**

| | Content of succinic acid (wt%) | Content of SO₄²⁻ (ppm) | Content of a phosphorus element (ppm) |
|---|---|---|---|
| Example 1 | 99.81 | 412 | 19.4 |
| Example 2 | 99.76 | 377 | 16.5 |
| Example 3 | 99.71 | 322 | 22.3 |
| Example 4 | 99.71 | 395 | 8.6 |
| Example 5 | 99.70 | 462 | 4.5 |
| Example 6 | 99.68 | 280 | 35.5 |
| Comparative Example 1 | 99.72 | 96 | 0.2 |
| Comparative Example 2 | 99.53 | 550 | 48.2 |
| Comparative Example 3 | 99.74 | 395 | 0 |
| Comparative Example 4 | 99.75 | 47 | 8.6 |
| Comparative Example 5 | 99.78 | 47 | 0 |

Methods for preparing the succinic acid compositions provided in Examples 1-3, Example 5, Example 6, and Comparative Example 2 are as follows.

### Example 1

This example provides a succinic acid composition. A method for preparing the succinic acid composition includes:
(1) using a biomass resource as a raw material to prepare and obtain a succinic acid fermentation solution by a fermentation method, where the fermentation solution is prepared by a conventional method in the art, and for example, may be prepared by referring to a method in CN118459331A; and after removing cells from the fermentation solution by filtration, adding an excessive amount of calcium hydroxide into a fermentation supernatant until no more calcium succinate precipitate is produced in the succinic acid fermentation solution, and then performing filtration and separation to obtain the calcium succinate precipitate;
(2) adding concentrated sulfuric acid into the calcium succinate obtained in the step (1) for acidification, and adjusting the pH of the system to 2.3 to obtain a succinic acid stock solution with a succinic acid content of 72 g/L;
(3) based on a volume of 1 L of the succinic acid stock solution obtained in the step (2), adding 26 g of diisooctyl phosphate for extraction 3 times to obtain a succinic acid-loaded organic phase; and then, based on a volume of 1 L of the succinic acid-loaded organic phase, subjecting the succinic acid-loaded organic phase to 3-stage reverse extraction with 55 g of deionized water to obtain an aqueous solution of succinic acid;
(4) allowing the aqueous solution of succinic acid obtained in the step (3) to pass through a 732-type cation exchange resin column at a flow rate of 1.6 BV/h, and performing elution with water with a mass 3 times that of the aqueous solution of succinic acid to obtain a succinic acid effluent;
(5) subjecting the succinic acid effluent obtained in the step (4) to decolorization with activated carbon at 75°C for 60 min, where based on a volume of 1 L of the succinic acid effluent, a mass of the activated carbon is 8 g (that is, the mass of the activated carbon is 8 g/L); and then, performing ultrafiltration at 50°C using an ultrafiltration membrane with a pore size of 40 nm to obtain a succinic acid filtrate; and
(6) subjecting the succinic acid filtrate obtained in the step (5) to reduced-pressure distillation treatment at a distillation temperature of 70°C and a pressure of -0.07 - -0.1 MPa, stopping the distillation when the content of succinic acid is greater than 15 wt%, and subjecting the concentrated succinic acid solution to cooling crystallization at a low temperature (4°C) to obtain a white succinic acid crystal, thereby obtaining the succinic acid composition.

### Example 2

This example provides a succinic acid composition, where steps (1) and (2) in its preparation method are the same as those in Example 1, with the differences as follows: (3) based on a volume of 1 L of the succinic acid stock solution obtained in the step (2), adding 26 g of diisooctyl phosphate for extraction 4 times to obtain a succinic acid-loaded organic phase; and then, based on a volume of 1 L of the succinic acid-loaded organic phase, subjecting the succinic acid-loaded organic phase to 4-stage reverse extraction with 70 g of deionized water to obtain an aqueous solution of succinic acid;
(4) allowing the aqueous solution of succinic acid obtained in the step (3) to pass through a 732-type cation exchange resin column at a flow rate of 1.5 BV/h, and performing elution with water with a mass 4 times that of the aqueous solution of succinic acid to obtain a succinic acid effluent;
(5) subjecting the succinic acid effluent obtained in the step (4) to decolorization with activated carbon at 75°C for 60 min, where based on a volume of 1 L of the succinic acid effluent, a mass of the activated carbon is 8 g (that is, the mass of the activated carbon is 8 g/L); and then, performing ultrafiltration at 50°C using an ultrafiltration membrane with a pore size of 40 nm to obtain a succinic acid filtrate; and
(6) subjecting the succinic acid filtrate obtained in the step (5) to reduced-pressure distillation treatment at a distillation temperature of 70°C and a pressure of -0.07 - -0.1 MPa, stopping the distillation when the content of succinic acid is greater than 15 wt%, and subjecting the concentrated succinic acid solution to cooling crystallization at a low temperature (4°C) to obtain a white succinic acid crystal, thereby obtaining the succinic acid composition.

### Example 3

This example provides a succinic acid composition, where steps (1) and (2) in its preparation method are the same as those in Example 1, with the differences as follows:
(3) based on a volume of 1 L of the succinic acid stock solution obtained in the step (2), adding 17 g of diethyl phosphate for extraction 3 times to obtain a succinic acid-loaded organic phase; and then, based on a volume of 1 L of the succinic acid-loaded organic phase, subjecting the succinic acid-loaded organic phase to 3-stage reverse extraction with 65 g of deionized water to obtain an aqueous solution of succinic acid;
(4) allowing the aqueous solution of succinic acid obtained in the step (3) to pass through a 732-type cation exchange resin column at a flow rate of 1.4 BV/h, and performing elution with water with a mass 5 times that of the aqueous solution of succinic acid to obtain a succinic acid effluent;
(5) subjecting the succinic acid effluent obtained in the step (4) to decolorization with activated carbon at 70°C for 60 min, where based on a volume of 1 L of the succinic acid effluent, a mass of the activated carbon is 7 g (that is, the mass of the activated carbon is 7 g/L); and then, performing ultrafiltration at 50°C using an ultrafiltration membrane with a pore size of 40 nm to obtain a succinic acid filtrate; and
(6) subjecting the succinic acid filtrate obtained in the step (5) to reduced-pressure distillation treatment at a distillation temperature of 70°C and a pressure of -0.07 - -0.1 MPa, stopping the distillation when the content of succinic acid is greater than 15 wt%, and subjecting the concentrated succinic acid solution to cooling crystallization at a low temperature (4°C) to obtain a white succinic acid crystal, thereby obtaining the succinic acid composition.

### Example 5

This example provides a succinic acid composition, where a step (1) in its preparation method is the same as that in Example 1, with the differences as follows:
(2) adding concentrated sulfuric acid into the calcium succinate obtained in the step (1) for acidification, and adjusting the pH of the system to 1.6 to obtain a succinic acid stock solution with a succinic acid content of 68 g/L;
(3) based on a volume of 1 L of the succinic acid stock solution obtained in the step (2), adding 5 g of triethyl phosphate for extraction 2 times to obtain a succinic acid-loaded organic phase; and then, based on a volume of 1 L of the succinic acid-loaded organic phase, subjecting the succinic acid-loaded organic phase to 2-stage reverse extraction with 50 g of deionized water to obtain an aqueous solution of succinic acid;
(4) allowing the aqueous solution of succinic acid obtained in the step (3) to pass through a 732-type cation exchange resin column at a flow rate of 1.7 BV/h, and performing elution with water with a mass 4 times that of the aqueous solution of succinic acid to obtain a succinic acid effluent;
(5) subjecting the succinic acid effluent obtained in the step (4) to decolorization with activated carbon at 70°C for 60 min, where based on a volume of 1 L of the succinic acid effluent, a mass of the activated carbon is 8 g (that is, the mass of the activated carbon is 8 g/L); and then, performing ultrafiltration at 50°C using an ultrafiltration membrane with a pore size of 40 nm to obtain a succinic acid filtrate; and
(6) subjecting the succinic acid filtrate obtained in the step (5) to reduced-pressure distillation treatment at a distillation temperature of 70°C and a pressure of -0.07 - -0.1 MPa, stopping the distillation when the content of succinic acid is greater than 15 wt%, and subjecting the concentrated succinic acid solution to cooling crystallization at a low temperature (4°C) to obtain a white succinic acid crystal, thereby obtaining the succinic acid composition.

### Example 6

This example provides a succinic acid composition, where a step (1) in its preparation method is the same as that in Example 1, with the differences as follows:
(2) adding concentrated sulfuric acid into the calcium succinate obtained in the step (1) for acidification, and adjusting the pH of the system to 2.7 to obtain a succinic acid stock solution with a succinic acid content of 77 g/L;
(3) based on a volume of 1 L of the succinic acid stock solution obtained in the step (2), adding 21 g of diethyl phosphate for extraction 3 times to obtain a succinic acid-loaded organic phase; and then, based on a volume of 1 L of the succinic acid-loaded organic phase, subjecting the succinic acid-loaded organic phase to 2-stage reverse extraction with 60 g of deionized water to obtain an aqueous solution of succinic acid;
(4) allowing the aqueous solution of succinic acid obtained in the step (3) to pass through a 732-type cation exchange resin column at a flow rate of 1.4 BV/h, and performing elution with water with a mass 6 times that of the aqueous solution of succinic acid to obtain a succinic acid effluent;
(5) subjecting the succinic acid effluent obtained in the step (4) to decolorization with activated carbon at 75°C for 70 min, where based on a volume of 1 L of the succinic acid effluent, a mass of the activated carbon is 8 g (that is, the mass of the activated carbon is 8 g/L); and then, performing ultrafiltration at 50°C using an ultrafiltration membrane with a pore size of 40 nm to obtain a succinic acid filtrate; and
(6) subjecting the succinic acid filtrate obtained in the step (5) to reduced-pressure distillation treatment at a distillation temperature of 70°C and a pressure of -0.07 - -0.1 MPa, stopping the distillation when the content of succinic acid is greater than 15 wt%, and subjecting the concentrated succinic acid solution to cooling crystallization at a low temperature (4°C) to obtain a white succinic acid crystal, thereby obtaining the succinic acid composition.

### Comparative Example 2

This comparative example provides a succinic acid composition, where a step (1) in its preparation method is the same as that in Example 1, with the differences as follows:
(2) introducing concentrated sulfuric acid into the calcium succinate obtained in the step (1) for acidification, and adjusting the pH of the system to 1.1 to obtain a succinic acid stock solution with a succinic acid content of 68 g/L;
(3) based on a volume of 1 L of the succinic acid stock solution obtained in the step (2), adding 30 g of diethyl phosphate for extraction 3 times to obtain a succinic acid-loaded organic phase; and then, based on a volume of 1 L of the succinic acid-loaded organic phase, subjecting the succinic acid-loaded organic phase to 2-stage reverse extraction with 60 g of deionized water to obtain an aqueous solution of succinic acid;
(4) allowing the aqueous solution of succinic acid obtained in the step (3) to pass through a 732-type cation exchange resin column at a flow rate of 1.6 BV/h, and performing elution with water with a mass 3 times that of the aqueous solution of succinic acid to obtain a succinic acid effluent;
(5) subjecting the succinic acid effluent obtained in the step (4) to decolorization with activated carbon at 70°C for 55 min, where based on a volume of 1 L of the succinic acid effluent, a mass of the activated carbon is 8 g (that is, the mass of the activated carbon is 8 g/L); and then, performing ultrafiltration at 50°C using an ultrafiltration membrane with a pore size of 40 nm to obtain a succinic acid filtrate; and
(6) subjecting the succinic acid filtrate obtained in the step (5) to reduced-pressure distillation treatment at a distillation temperature of 70°C and a pressure of -0.07 - -0.1 MPa, stopping the distillation when the content of succinic acid is greater than 15 wt%, and subjecting the concentrated succinic acid solution to cooling crystallization at a low temperature (4°C) to obtain a white succinic acid crystal, thereby obtaining the succinic acid composition.

### Application Example 1

This application example provides a polyester, where raw materials for preparing the polyester include 350 kg of butanediol, 340 kg of succinic acid, 1.2 kg of glycerol, and 0.47 kg of tetrabutyl titanate; and the succinic acid is the succinic acid composition provided in Example 1. A method for preparing the polyester includes the following steps:
(1) physically mixing the succinic acid, the 1,4-butanediol, and the glycerol, and after the mixing is completed, transferring a resulting mixture into an esterification reactor to enable the reaction mixture to carry out an esterification reaction at a temperature of 180°C and a pressure of 1.0 bar for 3 h to obtain an esterification product;
(2) transferring the esterification product obtained in the step (1) into a vertical reactor with a stirrer, adding the tetrabutyl titanate, and carrying out a pre-polycondensation reaction at a temperature of 245°C and a pressure of 0.36 bar for 85 min to obtain a pre-polycondensation product; and
(3) transferring the pre-polycondensation obtained in the step (2) into a horizontal reactor with a stirrer to carry out a polycondensation reaction at a temperature of 248°C and a pressure of 2.0 mbar for 150 min, and performing slicing and drying to obtain the polyester.

### Application Examples 2-6

Application Examples 2-6 respectively provide a polyester, where the only difference from Application Example 1 is that the succinic acid is the succinic acid composition provided in Examples 2-6, respectively. Other raw materials, use amounts, and preparation methods are all the same as those in Application Example 1.

### Application Example 7

Application Example 7 provides a polyester, where the differences from Application Example 1 are that with a total molar amount of the dicarboxylic acids remaining unchanged, the polyester includes 75 mol% of succinic acid and 25 mol% of sebacic acid, and the succinic acid is the succinic acid composition provided in Example 1; and raw materials for preparing the polyester further include 350 kg of butanediol, 2.2 kg of glycerol, and 0.5 kg of tetrabutyl titanate. A method for preparing the polyester includes the following steps:
(1) physically mixing the succinic acid, the sebacic acid, the 1,4-butanediol, and the glycerol, and after the mixing is completed, transferring a resulting mixture into an esterification reactor to enable the reaction mixture to carry out an esterification reaction at a temperature of 210°C and a pressure of 1.2 bar for 5 h to obtain an esterification product;
(2) transferring the esterification product obtained in the step (1) into a vertical reactor with a stirrer, adding the tetrabutyl titanate, and carrying out a pre-polycondensation reaction at a temperature of 255°C and a pressure of 0.56 bar for 100 min to obtain a pre-polycondensation product; and
(3) transferring the pre-polycondensation obtained in the step (2) into a horizontal reactor with a stirrer to carry out a polycondensation reaction at a temperature of 250°C and a pressure of 1.0 mbar for 125 min, and performing slicing and drying to obtain the polyester.

### Application Example 8

Application Example 8 provides a polyester, where the differences from Application Example 1 are that with a total molar amount of the dicarboxylic acids remaining unchanged, the polyester includes 68 mol% of succinic acid and 32 mol% of sebacic acid, and the succinic acid is the succinic acid composition provided in Example 1; and raw materials for preparing the polyester further include 350 kg of butanediol, 2.5 kg of glycerol, and 0.55 kg of tetrabutyl titanate. A method for preparing the polyester includes the following steps:
(1) physically mixing the succinic acid, the sebacic acid, the 1,4-butanediol, and the glycerol, and after the mixing is completed, transferring a resulting mixture into an esterification reactor to enable the reaction mixture to carry out an esterification reaction at a temperature of 200°C and a pressure of 1.8 bar for 2 h to obtain an esterification product;
(2) transferring the esterification product obtained in the step (1) into a vertical reactor with a stirrer, adding the tetrabutyl titanate, and carrying out a pre-polycondensation reaction at a temperature of 255°C and a pressure of 0.3 bar for 110 min to obtain a pre-polycondensation product; and
(3) transferring the pre-polycondensation obtained in the step (2) into a horizontal reactor with a stirrer to carry out a polycondensation reaction at a temperature of 255°C and a pressure of 1.5 mbar for 130 min, and performing slicing and drying to obtain the polyester.

### Application Example 9

Application Example 9 provides a polyester, where the only difference from Application Example 1 is that with a total molar amount of the dicarboxylic acids remaining unchanged, the polyester includes 75 mol% of succinic acid and 25 mol% of adipic acid. Other raw materials, use amounts, and a preparation method are all the same as those in Application Example 1.

### Comparative Application Examples 1-5

Comparative Application Examples 1-5 respectively provide a polyester, where the only difference from Application Example 1 is that the succinic acid is the succinic acid composition provided in Comparative Examples 1-4 and the succinic acid provided in Comparative Example 5, respectively. Other raw materials, use amounts, and preparation methods are all the same as those in Application Example 1.

The acid number, viscosity, SO₄²⁻ content, and phosphorus element content of the polyesters provided in Application Examples 1-9 and Comparative Application Examples 1-5 were tested, respectively.

A method for testing the acid number includes: determining the acid number (AN, mg KOH/g) of a sample according to the DIN EN 12634 standard in October, 1998.

A solvent mixture used includes: 1 part by volume of dimethyl sulfoxide, 8 parts by volume of isopropanol, and 7 parts by volume of toluene, where a total volume of the solvent mixture was 150 mL.

According to the provisions of the DIN EN 12634 standard, samples were pre-titrated to determine appropriate sample masses and ensure that a volume of a titration solution consumed was 2-3 mL. The samples were added into the solvent mixture and heated to 70-85°C to ensure that all the samples were completely dissolved into clear solutions, and during the titration, the temperatures of the solutions were maintained at 65-75°C to avoid precipitation of the samples. If appropriate, tetrabutylammonium hydroxide was selected as the titration solution while avoiding the use of highly toxic tetramethylammonium hydroxide. Meanwhile, to prevent the solvent mixture from absorbing CO₂ in the air and affecting the volume of the titration solution consumed by a blank solvent, when the volume of the titration solution consumed by the blank solvent was tested, the blank solvent was pre-treated in the same test operation process as the samples. For example, the blank solvent was subjected to heating treatment at the same time and temperature, and then the blank solvent was titrated.

A method for testing the viscosity includes: according to the provisions of GB/T 17931-1999, measuring the viscosity in a phenol/o-dichlorobenzene solution at a weight ratio of 1:1 in a thermostatic water bath at 25±0.05°C, where a sample concentration was 5 mg/mL.

Methods for testing the SO₄²⁻ content and the phosphorus element content are as previously described.

Specific test results are shown in Table 2, where ND represents undetected.

**Table 2**

| | Acid number (mg KOH/g) | Viscosity (dL/g) | Content of SO₄²⁻ (ppm) | Content of a phosphorus element (ppm) |
|---|---|---|---|---|
| Application Example 1 | 0.85 | 1.64 | 187 | 7.4 |
| Application Example 2 | 0.79 | 1.67 | 162 | 6.1 |
| Application Example 3 | 1.0 | 1.52 | 137 | 8.2 |
| Application Example 4 | 0.76 | 1.68 | 170 | 3.9 |
| Application Example 5 | 0.96 | 1.57 | 217 | 2.5 |
| Application Example 6 | 1.18 | 1.45 | 105 | 14.2 |
| Application Example 7 | 0.82 | 1.57 | 125 | 5.5 |
| Application Example 8 | 0.80 | 1.53 | 111 | 4.4 |
| Application Example 9 | 0.73 | 1.63 | 126 | 5.2 |
| Comparative Application Example 1 | 1.72 | 1.33 | 37 | ND |
| Comparative Application Example 2 | 1.24 | 1.42 | 267 | 26.8 |
| Comparative Application Example 3 | 0.72 | 1.66 | 174 | ND |
| Comparative Application Example 4 | 1.89 | 1.28 | ND | 3.7 |
| Comparative Application Example 5 | 2.12 | 1.20 | ND | ND |

It can be seen from Table 2 that the polyester prepared using the succinic acid composition provided by the present invention as a raw material has a low acid number and a high viscosity; and the polyester has an acid number of ≤ 1.18 mg KOH/g and a viscosity of ≥ 1.45 dL/g.

It can be seen from the comparative application examples that the polyester with both a low acid number and a high viscosity cannot be obtained simultaneously when the content of the sulfate radical and the content of the phosphorus element in the succinic acid composition are not within specified ranges.

### Application Examples 2-1 - 2-12 and Comparative Application Examples 2-1 - 2-5

Application Examples 2-1 - 2-12 and Comparative Application Examples 2-1 - 2-5 respectively provide a polyester composite material. In parts by weight, formulations of the polyester composite materials are shown in Table 3 and Table 4, where "/" represents absence of the component in the formulation.

**Table 3**

| | | Application Example 2-1 | Application Example 2-2 | Application Example 2-3 | Application Example 2-4 | Application Example 2-5 | Application Example 2-6 | Application Example 2-7 | Application Example 2-8 | Application Example 2-9 |
|---|---|---|---|---|---|---|---|---|---|---|
| Polyes ter | Applicati on Example 1 | 58.5 | / | / | / | / | / | / | / | / |
| | Applicati on Example 2 | / | 58.5 | / | / | / | / | / | / | / |
| | Applicati on Example 3 | / | / | 58.5 | / | / | / | / | / | / |
| | Applicati on Example 4 | / | / | / | 58.5 | / | / | / | / | / |
| | Applicati on Example 5 | / | / | / | / | 58.5 | / | / | / | / |
| | Applicati on Example 6 | / | / | / | / | / | 58.5 | / | / | / |
| | Applicati on Example 7 | / | / | / | / | / | / | 58.5 | / | / |
| | Applicati on Example 8 | / | / | / | / | / | / | / | 58.5 | / |
| | Applicati on Example 9 | / | / | / | / | / | / | / | / | 58.5 |
| | Compara tive Applicati on Example 1 | / | / | / | / | / | / | / | / | / |
| | Compara tive Applicati on Example 2 | / | / | / | / | / | / | / | / | / |
| | Compara tive Applicati on Example 3 | / | / | / | / | / | / | / | / | / |
| | Compara tive Applicati on Example 4 | / | / | / | / | / | / | / | / | / |
| | Compara tive Applicati on Example 5 | / | / | / | / | / | / | / | / | / |
| Polylactic acid | | 25 | 25 | 25 | 25 | 25 | 25 | 25 | 25 | 25 |
| Filler | Talc powder | 12 | 12 | 12 | 12 | 12 | 12 | 12 | 12 | 12 |
| | Calcium carbonate | 4 | 4 | 4 | 4 | 4 | 4 | 4 | 4 | 4 |
| Auxiliary agent | Antioxid ant 1010 | 0.5 | 0.5 | 0.5 | 0.5 | 0.5 | 0.5 | 0.5 | 0.5 | 0.5 |

**Table 4**

| | | Applicati on Example 2-10 | Applicati on Example 2-11 | Applicati on Example 2-12 | Comparat ive Applicati on Example 2-1 | Comparat ive Applicati on Example 2-2 | Comparat ive Applicati on Example 2-3 | Comparat ive Applicati on Example 2-4 | Comparat ive Applicati on Example 2-5 |
|---|---|---|---|---|---|---|---|---|---|
| Polyest er | Applicati on Example 1 | 46.8 | 64.2 | 58.5 | / | / | / | / | / |
| | Applicati on Example 2 | / | / | / | / | / | / | / | / |
| | Applicati on Example 3 | / | / | / | / | / | / | / | / |
| | Applicati on Example 4 | / | / | / | | / | / | / | / |
| | Applicati | / | / | / | / | / | / | / | / |
| | on Example 5 | | | | | | | | |
| | Applicati on Example 6 | / | / | / | / | / | / | / | / |
| | Applicati on Example 7 | / | / | / | / | / | / | / | / |
| | Applicati on Example 8 | / | / | / | / | / | / | / | / |
| | Applicati on Example 9 | / | / | / | / | / | / | / | / |
| | Comparat ive Applicati on Example 1 | / | / | / | 58.5 | / | / | / | / |
| | Comparat ive Applicati on Example 2 | / | / | / | / | 58.5 | / | / | / |
| | Comparat ive Applicati on | / | / | / | / | / | 58.5 | / | / |
| | Example 3 | | | | | | | | |
| | Comparat ive Applicati on Example 4 | / | / | / | / | / | / | 58.5 | / |
| | Comparat ive Applicati on Example 5 | / | / | / | / | / | / | / | 58.5 |
| Polylactic acid | | 29 | 23 | 25 | 25 | 25 | 25 | 25 | 25 |
| Filler | Talc powder | 18 | 9 | 16 | 12 | 12 | 12 | 12 | 12 |
| | Calcium carbonate | 6 | 3 | / | 4 | 4 | 4 | 4 | 4 |
| Auxilia ry agent | Antioxida nt 1010 | 0.2 | 0.8 | 0.5 | 0.5 | 0.5 | 0.5 | 0.5 | 0.5 |

### Performance test

Before processing, surfaces of screws of an extruder were cleaned with cotton cloth, and diameters at middle positions of the screws were each measured in parallel three times using a caliper to obtain a mean value, designated as D₀ (unit: mm). After the polyester composite materials provided in Application Examples 2-1 - 2-12 and Comparative Application Examples 2-1 - 2-5 were continuously produced in the twin-screw extruder for 168 hours, respectively, the surfaces of the screws were cleaned with cotton cloth, and diameters at the same positions as those before processing were each measured in parallel three times using a caliper to obtain a mean value, designated as D₁ (unit: mm). The degree of corrosion of the screws of the twin-screw extruder was characterized by a change in the diameters of the screws before and after processing, and a wear percentage η of the screws was calculated as η=(ΔD/D₀)×100%, where ΔD=D₀-D₁, in the unit of mm. The greater wear percentage η of the screws indicates more severe corrosion of the screws.

Process parameters of the twin-screw extruder are as follows:
temperatures of different sections being sequentially as follows: 170°C-180°C-180°C-180°C-190°C-190°C-200°C-200°C-200°C-180°C; feeding speed: 800 kg/h; and vacuum pressure: -0.07 - -0.1 MPa.

Specific test results are shown in Table 5.

**Table 5**

| | η/% |
|---|---|
| Application Example 2-1 | 0.19 |
| Application Example 2-2 | 0.08 |
| Application Example 2-3 | 0.39 |
| Application Example 2-4 | 0.13 |
| Application Example 2-5 | 0.40 |
| Application Example 2-6 | 0.48 |
| Application Example 2-7 | 0.18 |
| Application Example 2-8 | 0.16 |
| Application Example 2-9 | 0.16 |
| Application Example 2-10 | 0.29 |
| Application Example 2-11 | 0.16 |
| Application Example 2-12 | 0.18 |
| Comparative Application Example 2-1 | 0.73 |
| Comparative Application Example 2-2 | 0.55 |
| Comparative Application Example 2-3 | 0.56 |
| Comparative Application Example 2-4 | 0.82 |
| Comparative Application Example 2-5 | 1.18 |

It can be seen from Table 5 that after the polyester composite material formed from the polyester prepared using the succinic acid composition provided by the present invention as a raw material is continuously produced in the twin-screw extruder for 168 hours, the wear percentage of the screws of the twin-screw extruder is less than or equal to 0.5%, which can significantly reduce the corrosion of metal equipment caused by the polyester material.

However, after the polyester compositions provided in the comparative application examples are continuously produced for 168 hours, the diameters of the screws are obviously changed, and the corrosion of equipment caused by the polyester composite materials is relatively severe.

The specific examples described above are to further describe the purposes, technical solutions, and beneficial effects of the present invention in detail. It should be understood that the above descriptions are only specific examples of the present invention and are not intended to limit the present invention, and any modifications, equivalent substitutions, improvements, and the like made within the spirit and principles of the present invention should all be included within the scope of protection of the present invention.

## Claims

1. A succinic acid composition, comprising succinic acid, a compound containing a sulfate radical, and a phosphorus compound, wherein
a mass content of the sulfate radical in the succinic acid composition is 240-500 ppm; and
a mass content of a phosphorus element in the succinic acid composition is 0.5-40 ppm.

2. The succinic acid composition according to claim 1, wherein the mass content of the sulfate radical in the succinic acid composition is 320-480 ppm, preferably 370-420 ppm.

3. The succinic acid composition according to claim 1 or 2, wherein the mass content of the phosphorus element in the succinic acid composition is 2-25 ppm, more preferably 5-20 ppm.

4. The succinic acid composition according to any one of claims 1-3, wherein the compound containing the sulfate radical comprises sulfuric acid and/or a sulfate;
preferably, the sulfate comprises any one or at least two of sodium sulfate, magnesium sulfate, potassium sulfate, or ferric sulfate;
preferably, the phosphorus compound comprises any one or at least two of nucleic acid, phospholipid, diisooctyl phosphate, diethyl phosphate, triethyl phosphate, sodium dihydrogen phosphate, disodium hydrogen phosphate, phosphoric acid, phosphorous acid, or sodium phosphate; and
preferably, a mass percentage content of the succinic acid in the succinic acid composition is ≥ 99.5%, further preferably ≥ 99.7%.

5. A polyester, wherein raw materials for preparing the polyester comprise the succinic acid composition according to any one of claims 1-4.

6. The polyester according to claim 5, comprising the following components:
component A:
based on a total molar amount of 100 mol% of the component A, being a dicarboxylic acid compound comprising the following components:
a1, being 65-100 mol% of succinic acid and/or a derivative of an ester thereof, and
a2, being 0-35 mol% of a C₆-C₁₄ dicarboxylic acid and/or a derivative of an ester thereof;
and
component B: being 1,4-butanediol with a molar amount at least equal to that of the component A, wherein
the succinic acid is the succinic acid composition according to any one of claims 1-4.

7. The polyester according to claim 5 or 6, wherein in the polyester, a mass content of the sulfate radical is 90-210 ppm; and
preferably, a mass content of the phosphorus element in the polyester is 0.1-15 ppm.

8. A polyester composite material, wherein the polyester composite material is a composite material obtained by extrusion processing; and
the polyester composite material comprises the polyester according to any one of claims 5-7.

9. The polyester composite material according to claim 8, wherein further comprising at least one of a polyhydroxyalkanoate, an inorganic filler, or an auxiliary agent.

10. The polyester composite material according to claim 8 or 9, wherein when the polyester composite material is continuously produced in a twin-screw extruder for 168 hours, a wear percentage of screws is ≤ 0.5%.
